Europäisches Patentamt

European Patent Office

Office européen des brevets

(11) Publication number: **0 127 476**
**B1**

# EUROPEAN PATENT SPECIFICATION

(45) Date of publication of patent specification: **28.11.90**

(21) Application number: **84303583.3**

(22) Date of filing: **25.05.84**

(51) Int. Cl.⁵: **G 01 D 5/26,** G 01 L 11/00,
A 61 B 5/02

(54) Fibre-optic transducer apparatus.

(30) Priority: **25.05.83 US 498092**

(43) Date of publication of application:
**05.12.84 Bulletin 84/49**

(45) Publication of the grant of the patent:
**28.11.90 Bulletin 90/48**

(84) Designated Contracting States:
**AT BE CH DE FR GB IT LI NL SE**

(56) References cited:
**DE-A-2 849 186**
**DE-A-2 905 630**
**US-A-3 068 739**
**US-A-4 358 960**

(73) Proprietor: **CAMINO LABORATORIES, INC.**
**5955 Pacific Center Boulevard**
**San Diego California 92121 (US)**

(72) Inventor: **Knute, Wallace L.**
**1050 Solana Drive**
**Del Mar, California 92014 (US)**
Inventor: **Bailey, Wilber H.**
**1015 Hymettus Avenue**
**Leucadia, California 92024 (US)**

(74) Representative: **Howden, Christopher Andrew**
**FORRESTER & BOEHMERT Widenmayerstrasse**
**4/I**
**D-8000 München 22 (DE)**

## Description

This invention relates generally to transducer systems for measuring physical variables and, more particularly, to systems of this kind that transmit a modulated light beam along an optical fiber.

Optical transducer systems of this particular kind have been used in the past in the medical field for such applications as measuring a patient's blood pressure, intra-cranial pressure, body temperature, etc. The typical system includes a catheter comprised of two sets of optical fibers located within a resilient sheath, which is adapted for insertion into a patient's body. One set of fibers transmits a light beam to a transducer located at the remote end of the catheter, and the other set of fibers returns a modulated light beam from the transducer. The transducer modulates the beam by reflecting to the second set of fibers a proportion of the light beam that varies in accordance with the variable being measured. A photosensor senses the intensity of the returned light beam, to produce a signal indicative of the variable.

Fiber-optic transducer systems such as the one described above have not proven to be entirely satisfactory, and have not as yet met with widespread acceptance. One reason for this apparent lack of acceptability is believed to be that bending of the catheter can affect the intensity of the light beam returned from the transducer. The user cannot be sure whether a particular drop in signal level is due to an actual change in the variable being measured or to bending of the catheter. In addition, movement of the catheter can modulate the intensity of the returned light beam and thereby make it difficult to measure the variable accurately.

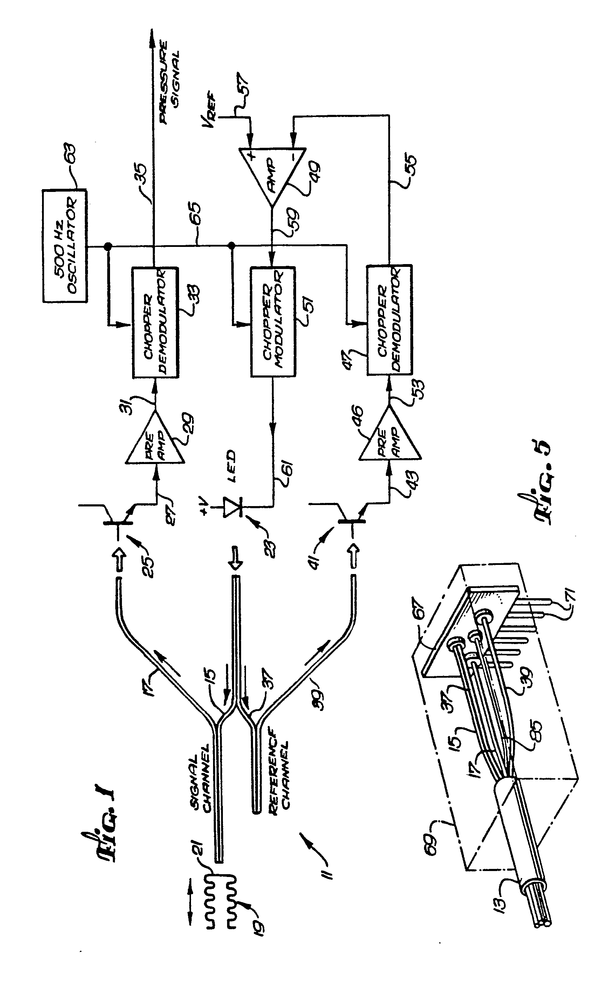

German Patent Specification DE—A—2849186 from which the preamble of present Claim 1 has been derived discloses optical transducer apparatus in which a measurement light beam is transmitted from a light source by first optical fiber means to transducing means located at a remote location and acting to intensity modulate the measurement light beam in accordance with the magnitude of a variable to be measured. The light source is intermittently energised by a modulator at a rate determined by an oscillator and the measurement light beam is returned by the first optical fiber means to photosensing means to produce a correspondingly intermittent measurement signal. Correcting means synchronized with the modulator and connected to the light source adjusts the intensity of the measurement light beam to correct the measurement signal for variations in the transmittance of the first optical fiber means. The correcting means includes second optical fiber means which serve to transmit a reference light beam to reflecting means of the transducing means and the photosensing means provides a signal indicative of the intensity of the reference light beam which is returned from the reflecting means by the first optical fiber means. Moreover, Fig. 1b of this Specification discloses the principle of using a single light source generating both the measurement light beam and the reference light beam. The intensity of the light transmitted by said light source is continuously adjusted to be constant in response to the reference light beam received after reflection on a remote reflection means. The reference light beam is transmitted via a plurality of fibers. A first photosensing element produces the measurement signal while a second photosensing element responds to the said reflected reference light beam.

United states Patent Specification US—A—3068739 discloses a flexible optical probe, used as a catheter and having its optical fiber surrounded by a sheath.

There remains, however, a definite need for an improved fiber-optic transducer apparatus that is not susceptible to output signal variations caused by bending or movement of the fibers. The present invention fulfills these needs.

According to the present invention there is provided optical transducer apparatus comprising: a light source; first optical fiber means coupled to the light source for transmitting a measurement light beam to and from a remote location, such first optical fiber means comprising a plurality of fibers and having a variable light transmittance; transducing means located at the remote location for intensity modulating the light beam of the first optical fiber means in accordance with the magnitude of a predetermined variable to be measured; photosensing means for producing a measurement signal indicative of the intensity of the light beam transmitted from the remote location by the first optical fiber means; a modulator for intermittently energising the light source at a rate determined by an oscillator to produce a correspondingly intermittent measurement signal; and correcting means synchronized with the modulator and connected to the light source for adjusting the intensity of the measurement light beam to correct the measurement signal for variations in the transmittance of the first optical fiber means, the correcting means including second optical fiber means which serve to transmit a reference light beam to reflecting means at the remote location, the photosening means providing a signal indicative of the intensity of the reference light beam transmitted from the remote location; characterised in that: the first and second optical fiber means are surrounded by a sheath; the second optical fiber means comprises a plurality of fibers for transmitting the reference light beam to and from the reflecting means; the reflecting means is separated from the transducing means at the remote location and encloses the remote ends of the plurality of fibers of the second optical fiber means; the photosensing means comprises a first photosensing element for producing the measurement signal and a second photosensing element for producing the signal indicative of the intensity of the reference light beam returned by the second optical fiber means; and the correcting means continuously adjusts the intensity of the reference light beam and of the measurement light beam, the intensity of the reference light beam being adjusted in accordance with the signal from the second photosensing element so that the reference light beam returned by the second optical fiber means has a substantially constant intensity.

Apparatus embodying the invention enables accurate measurement of the variable being measured, even though the optical fiber means might be bent by an unknown amount.

In order that the invention may be more readily understood, an embodiment thereof will now be described, by way of example, with reference to the accompanying drawings, in which:

Figure 1 is a simplified schematic diagram of a pressure transducer apparatus embodying the present invention;

Figure 2 is a cross-sectional view of the remote end of the fiber-optic catheter of Figure 1, showing the remote ends of the optical fibers and a pressure-responsive bellows;

Figure 3 is a cross-sectional view of the catheter, taken in the direction of the arrows 3-3 in Figure 2;

Figure 4 is a cross-sectional view of the catheter, taken in the direction of the arrows 4-4 in Figure 2; and

Figure 5 is a perspective view of a portion of the transducer apparatus, depicting the near end of the fiber-optic catheter and the connections between its optical fibers and the light-emitting diode and phototransistors.

Description of the preferred embodiment

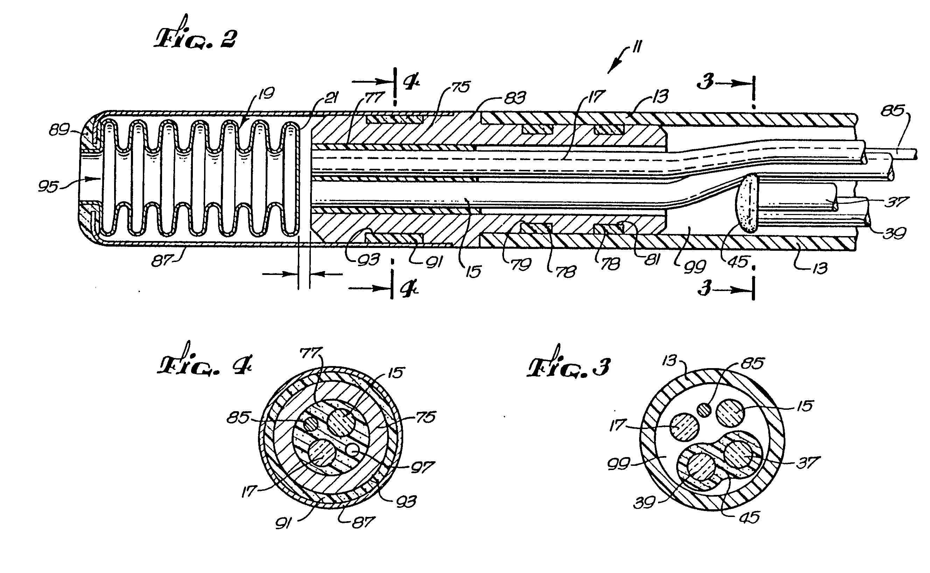

Referring now to the drawings, and particularly to Figs. 1 and 2, there is shown a fiber-optic pressure transducer adapted for insertion into a patient's vascular system to measure blood pressure. The apparatus includes a catheter 11 comprised of a polyurethane sheath 13 (Fig. 2) and a first set of optical fibers located within the sheath. The first set of fibers includes an emitter fiber 15 and a return fiber 17 for transmitting a first light beam to and from the catheter's remote end. A pressure transducer having a bellows 19 with a light-reflective surface 21 that moves in accordance with pressure is located at the catheter's remote end, for modulating the intensity of the first light beam.

A light-emitting diode (LED) 23 produces the light beam for transmission by the first set of optical fibers, and a first phototransistor 25 detects the modulated intensity of the returned beam. The phototransistor produces an intensity signal on line 27 having an electrical current that indicates the patient's blood pressure. This signal is amplified in a preamplifier 29 and coupled, in turn, on line 31 to a chopper demodulator 33, whose function is described below. The demodulator produce an analog pressure signal for output on line 35.

Since the catheter 11 is adapted for insertion into the patient's vein or artery, it necessarily experiences at least limited bending. This unfortunately affects the light transmittance of both the emitter fiber 15 and the return fiber 17, and therefore correspondingly affects the magnitude of the intensity signal produced by the first phototransistor 25. The light transmittance of the fibers can also vary with temperature. In addition, the efficiency of the LED 23 can vary with temperature and aging, and the efficiency of the phototransistor can vary with temperature. These variations result in corresponding changes in the intensity signal.

In accordance with the invention, the transducer apparatus further includes a reference channel comprised of a second set of optical fibers, located within the sheath 13 and extending substantially along its entire length. This reference channel is used to determine the effects of bending on transmittance and to determine the effects of temperature and aging on the efficiencies of the LED 23 and phototransistor 25. Since the first and second sets of optical fibers are substantially co-extensive and experience substantially the same degree of bending, the effect of that bending on their respective transmittances is presumed to be substantially equivalent. A second light beam produced by the LED 23 is transmitted by the second set optical fibers so that variations in the fibers' transmittance and variations in the LED's efficiency can be determined and a corresponding correction made to the intensity signal.

The second set of optical fibers includes an emitter fiber 37 for transmitting the second light beam from the LED 23 to a location near the end of the catheter 11 and a return fiber 39 for transmitting the light beam back from the location. A second phototransistor 41 detects the intensity of this returned light beam, to produce a correction signal on line 43 having an electrical current whose magnitude is proportional to intensity. The two phototransistors 25 and 41 are normally maintained at the same temperature, so their respective efficiencies tend to track each other.

A translucent droplet 45 of an epoxy containing a white pigment bonds together the remote ends of the two fibers 37 and 39 and thereby reflects a predetermined fixed proportion of the light beam from the emitter fiber to the return fiber. The epoxy droplet is preferably coated with an opaque silver paint, the opacity preventing movement of adjacent elements from affecting the return beam and the silver color maximizing the intensity of the return beam.

In the preferred embodiment, the second set of optical fibers is part of a feedback control system for regulating the current applied to the LED 23 so as to compensate for the variable light transmittance of the fibers and for the variable efficiencies of the LED 23 and phototransistors 25 and 41. Thus, if the transmittance of the second set of fibers decreases or if the efficiency of the LED decreases, for example, the control system automatically increases the drive current applied to the LED so that the intensity of the returned beam detected by the second phototransistor 41 remains substantially constant.

Besides the second set of optical fibers, the feedback control system includes the LED 23, the second phototransistor 41, a preamplifier 46, a chopper demodulator 47, a differential amplifier 49, and a chopper modulator 51. The correction signal produced by the second phototransistor is coupled on line 43 to the preamplifier for amplification, and coupling, in turn, on line 53 to the chopper demodulator, whose function is described below. The chopper demodulator outputs a signal proportional to the intensity of the returned second light beam for coupling on line 55 to the negative input terminal of the differential amplifier 49. A prescribed reference voltage is connected on line 57 to the amplifier's 49 positive input terminal. The resulting error signal is transmitted on line 59 from the differential amplifier 49 to the chopper modulator 51, whose function is described below, and in turn over line 61 to the LED 23.

The apparatus further includes chopper circuitry for preventing any dc offsets in the various circuit elements from affecting the pressure measurement being made. This chopper circuitry includes the previously-mentioned chopper demodulators 33 and 47 and chopper modulator 51, and in addition includes a 500 Hz oscillator 63, which produces a 500 Hz clock signal for coupling on line 65 to each demodulator and to the modulator. The modulator is essentially an analog gate for turning on and off the LED 23 at the 500 Hz rate, so that the electrical current signals produced by the first and second phototransistors 25 and 41, respectively, are modulated correspondingly. The two chopper demodulators are essentially sample-and-hold circuits for gating through the corresponding voltage signals only during those times when the LED is energized. As previously mentioned, this chopper circuitry ensures that any dc offsets in the various circuit elements do not affect the accuracy of the pressure measurement being made.

A perspective view of the coupling of the first and second sets of optical fibers to the LED 23 and phototransistors 25 and 41, respectively, is provided in Fig. 5. The electrical circuitry depicted schematically in Fig. 1 is mounted on a circuit board 67 located within a housing 69 and having a plurality of terminal pins 71 adapted for connection to additional processing circuitry (not shown). The various fibers of the first and second sets of optical fibers are optically coupled to the LED and phototransistors by means of a transparent optical gel potting material. In particular, each optical fiber is positioned with its end abutting the light-emitting surface of the LED or light-sensitive surface of the phototransistors. The potting material is deposited around the ends of the fibers, and a portion of its flows into any space located between the fibers and the surfaces. This provides an efficient coupling of light between the surfaces and their respective fibers.

The bellows 19 and the remote ends of the sheath 13 and the first and second sets of optical fibers are depicted in detail in Figs. 2, 4 and 5. The remote ends of the emitter fiber 15 and return fiber 17 of the first set are epoxied inside a stainless steel sleeve 75 that is coaxial with the sheath. The sleeve's inside diameter is slightly greater than the combined diameters of the two fibers (see Fig. 4), so that they readily fit within it. The epoxy, which is designated by the numeral 77, is drawn into the space between the fibers and the sleeve's inside wall for a distance of about one-half the sleeve's length. The remote ends of the two fibers are ground and polished flush with the far end of the sleeve, to provide an efficient optical coupling between the fibers and the light-reflective surface 21 of the bellows 19. This surface extends beyond the periphery of the sleeve opeving, as shown in Fig. 2, to accommodate all possible locations for the remote ends of the two fibers within the sleeve.

The remote end of the sheath 13 is expoxied to the outside surface of the sleeve 75. In particular, a mass of epoxy designated by the numeral 78 is located within a pair of channels 79 and 81 encircling the sleeve's outside surface. An annular flange 83 serves as a convenient stop for the sheath's remote end. The outside diameter of the flange is substantially equal to that of the sheath, i.e., about.049 inches (.124 cm), so as not to present any diameter discontinuity that might interfere with insertion of the catheter 11 into the patient's vein or artery.

The sheath 13 and the two fibers 15 and 17 of the first set of optical fibers have limited tensile strength and cannot, by themselves, withstand a substantial tensile stress. To alleviate this problem, the catheter 11 further includes a .006 inch (.015 cm) diameter wire 85 located within the sheath and connected between the circuit board 67 and the sleeve 75. It is attached to the inside wall of the sleeve by the epoxy material 77. The portion of the wire encased by the epoxy can have a slight bend or crimp in it to provide a stronger grip. The wire is preferably about one-eighth inch (.318 cm) shorter than the first set of optical fibers, so that the wire, and not the fibers, withstands any applied tensile stress. The additional fiber length is accounted for simply by a small amount of coiling within the sheath.

The remote ends of the emitter fiber 37 and return fiber 39 of the second set of optical fibers terminate just short of the near end of the sleeve 75, so as to be co-extensive with the first set of fibers for as long a distance as is practical. As previously mentioned, the fibers are bonded together by a translucent droplet 45 of epoxy, which reflects a predetermined proportion of the second light beam from the emitter fiber to the return fiber.

The pressure-sensitive bellows 19 is positioned a prescribed distance away from the remote ends of the first set of optical fibers by means of a stainless steel cover 87. In particular, the bellows is disposed within an opening in one end of the cover and secured in place by means of an epoxy material, designated by the numeral 89. the cover, in turn, is secured to the outside surface of the sleeve 75 by means of an epoxy material, designated by the numeral 91, located within channel 93 encircling the sleeve's outside surface. The annular flange 83 serves as a convenient stop for positioning the cover relative to the fibers 15 and 17 secured within the sleeve. The cover and flange have equal outside diameters.

The top surface 21 of the pressure-sensitive bellows 19, which confronts the remote ends of the emitter fibers 15 and return fiber 17 of the first set of optical fibers, is preferably plated with gold, to enhance reflectivity of the first light beam. This gold plating is preferably performed after the bellows has been epoxied to the cover 87, using an electroless process.

In the preferred embodiment, the emitter and return fibers 15 and 17 of the first set of optical fibers both have a diameter of .010 inches (.025 cm), and the lpht-reflective surface 21 of the bellows 19 is positioned about .003 inches (.008 cm) from their remote ends. At this spacing, relative axial movement of the bellows provides a high rate of change of optical coupling between the two fibers.

Several factors inherent in the manufacturing of the catheter 11 cause an uncertainty or inaccuracy in the relationship between the patient's actual blood pressure and the magnitude of the output pressure signal. These factors include uncertainties in the sensitivity of the bellows 19 and uncertainties in the relative positions of the remote ends of the two fibers 15 and 17 within the sleeve 75. Any such inaccuracy associated with the output pressure signal can be compensated for by appropriately calibrating each catheter. In particular, this can be accomplished by including special code circuitry (not shown) on the circuit board 67 associated with each catheter. An example of such code circuitry and of a system for utilizing it to provide a calibrated measurement signals is described in a copending and commonly-assigned U.S. patent application, Serial No. 385,492, filed in the name of Wilber H. Bailey and entitled "Transducer Calibration System."

In use, the catheter 11 is positioned in a patient's vein or artery and blood enters the hollow core 95 of the bellows 19, to press against the underside of its top wall. The opposite side of the top wall, i.e., the light-reflective surface 21 is vented to atmospheric pressure through a port 97 formed in the epoxy 77 located within the sleeve 75 and, in turn, through the empty space 99 within the sheath 13. The port 97 is formed by placing a wire through the sleeve when the epoxy is first inserted, and by removing the wire after the epoxy has hardened and the end of the fibers 15 and 17 have been ground and polished.

It should be appreciated from the foregoing description that the present invention provides an improved transducer apparatus having a fiber-optic catheter for particular use in medical applications such as invasive blood pressure and body temperature measurement. The apparatus includes a first set of optical fibers located within a sheath and transmitting a light beam to and from a remote transducer, which modulates the beam in accordance with the variable to be measured. A second set of optical fibers, which is likewise located within the sheath and is substantially co-extensive with the first set, is used to detect any variation in the light transmittance of the fibers caused by factors such as bending. The apparatus then controllably adjusts its measurement of the variable so as to correct for the effects of such varying transmittance.

Although the invention has been described in detail with reference to the presently-preferred embodiment, it should be understood by those of ordinary skill in the art that various modifications can be made without departing from the invention. Accordingly, the invention is limited only by the following claims.

## Claims

1. Optical transducer apparatus comprising: a light source (23); first optical fiber means (15, 17) coupled to the light source for transmitting a measurement light beam to and from a remote location, such first optical fiber means comprising a plurality of fibers and having a variable light transmittance; transducing means (19) located at the remote location for intensity modulating the light beam of the first optical fibers means in accordance with the magnitude of a predetermined variable to be measured; photosensing means (25, 41) for producing a measurement signal indicative of the intensity of the light beam transmitted from the remote location by the first optical fiber means; a modulator (51) for intermittently energising the light source at a rate determined by an oscillator (63) to produce a correspondingly intermittent measurement signal; and correcting means (37, 39, 41, 45 to 47, 49) synchronised with the modulator (51) and connected to the light source (23) for adjusting the intensity of the measurement light beam to correct the measurement signal for variations in the transmittance of the first optical fiber means (15, 17), the correcting means including second optical fiber means (37, 39) which serve to transmit a reference light beam to reflecting means (45) at the remote location, the photosensing means (25, 41) providing a signal indicative of the intensity of the reference light beam transmitted from the remote location; characterised in that: the first and second optical fiber means are surrounded by a sheath (13); the second optical fiber means (37, 39) comprises a plurality of fibers for transmitting the reference light beam to and from the reflecting means (45); the reflecting means is separated from the transducing means (19) at the remote location and encloses the remote ends of the plurality of fibers of the second optical fiber means (37, 39); the photosensing means comprises a first photosensing element (25) for producing the measurement signal and a second photosensing element (41) for producing the signal indicative of the intensity of the reference light beam returned by the second optical fiber means (37, 39); and the correcting means (37, 39, 41, 45 to 47, 49) continuously adjusts the intensity of the reference light beam and of the measurement light beam, the intensity of the reference light beam being adjusted in accordance with the signal from the second photosensing element (41) so that the reference light beam returned by the second optical fiber means has a substantially constant intensity.

2. Optical transducer apparatus according to claim 1, wherein the predetermined variable to be measured is the pressure of a fluid; the transducing means includes a light-reflective diaphragm (21) that is movable in accordance with the pressure of the fluid; and the first optical fiber means includes an emitter fiber (15) for directing the light beam at the light-reflective diaphragm of the transducing means and a return fiber (17) for receiving light reflected by the diaphragm, the proportion of the light beam that is reflected by the diaphragm and received by the return fiber varying in accordance with movement of the diaphragm, whereby the measurement signal is indicative of the pressure of the fluid.

3. Optical transducer apparatus according to claim 1, wherein the first optical fiber means includes an emitter fiber (15) for transmitting the light beam to the remote location, and a return fiber (17) for transmitting the modulated light beam from the remote location to the first photosensing element; and limited bending of the first optical fiber means varies the transmittance of the emitter fiber and the return fiber.

· 4. Optical transducer apparatus according to claim 3, wherein the second optical fiber means includes an emitter fiber (37) for transmitting the reference light beam to the reflecting means and a return fiber (39) for returning light from the reflecting means, the reflecting means (45) directing a predetermined proportion of the light supplied by the emitter fiber to the return fiber.

5. Optical transducer apparatus according to claim 3 or 4, comprising rigid termination means (75) and attachment means (77, 78) for attaching the remote ends of the emitter fiber, return fiber and sheath to the rigid termination means; and a wire (85) located within the sheath and attached by the attachment means to the rigid termination means, the wire reducing the tensile stress applied to the emitter fiber, the return fiber and the sheath.

6. Optical transducer apparatus according to claim 5, wherein the rigid termination means includes a sleeve (75); and the attachment means includes an epoxy material (77) located within the sleeve.

7. Optical transducer apparatus according to claim 6, wherein the predetermined variable to be measured is the pressure of a fluid; the transducing means (19) includes a light-reflective diaphragm (21) having a first surface bounding a chamber (95) vented to atmosphere and a second, opposite surface communicating with the fluid; and the epoxy material (77) includes a port (97) for venting the chamber (95) bounded by the first surface of the light-reflective diaphragm.

8. Optical transducer apparatus according to claim 6 or 7, wherein the sleeve (75) is located beyond the remote ends of the emitter fiber (37) and the return fiber (39) of the second optical fiber means; and the reflecting means of the second optical fiber means includes translucent epoxy means (45) bonding together the remote ends of the emitter fiber (37) and the return fiber (39) of the second optical fiber means.

9. Optical transducer apparatus according to any one of claims 6 to 8, wherein the wire (85) located within the sheath (13) is rigidly secured to the inside surface of the sleeve (75) by the epoxy material (77), the wire being shorter in length than both the sheath and the first optical fiber means (15, 17) for withstanding any tensile stress applied to the apparatus.

10. Optical transducer apparatus according to any preceding claim, wherein the predetermined variable to be measured is the pressure of a fluid; and the transducing means includes a bellows (19) having a light-reflective diaphragm (21) confronting in spaced relationship the remote ends of the emitter fiber (15) and return fiber (17) of the first optical fiber means, the bellows being movable with respect to the emitter and return fibers in acccordance with the pressure of the fluid.

**Patentansprüche**

1. Optische Wandlervorrichtung, die umfaßt; eine Lichtquelle (23); erste optische Fibermittel (15, 17), die mit der Lichtquelle verbunden sind, zum Übertragen eines Meßlichtstrahles zu und von einer entfernten Stelle, wobei die ersten optischen Fibermittel mehrere Fiber umfassen und einen variablen Licht-Transmissionsfaktor haben, Wandlermittel (19), die an der entfernten Stelle angeordnet sind, zum Modulieren der Intensität des Lichtstrahls der ersten optischen Fibermittel entsprechend der Größe einer zu messenden vorbestimmten Variablen; lichtempfindliche Mittel (25, 41) zum Erzeugen eines Meßsignals, welches die Intensität desjenigen Lichtstrahles anzeigt, der von der entfernten Stelle mittels der ersten optischen Fibermittel übertragen wird; einen Modulator (51) zum intermittierenden Versorgen der Lichtquelle mit Energie, und zwar mit einer durch einen Oszillator (63) bestimmten Rate, um ein entsprechendes intermittierendes Meßsignal zu erzeugen; und Korrekturmittel (37, 39, 41, 45 bis 47, 49), die mit dem Modulator (51) synchronisiert und mit der Lichtquelle (23) verbunden sind, zum Einstellen der Intensität des Meßlichtstrahles, um das Meßsignal im Hinblick auf Veränderungen des Transmissionsgrades der ersten optischen Fibermittel (15, 17) korrigieren, wobei die Korrekturmittel zweite optische Fibermittel (37, 39) umfassen, die dazu dienen, einen Bezugslichtstrahl an Reflektiermittel (45) an der entfernten Stelle zu übertragen, wobei die lichtempfindlichen Mittel (25, 41) ein Signal abgeben, welches die Intensität des Bezugslichtstrahles der von der entfernten Stelle übertragen wird, anzeigt; dadurch gekennzeichnet, daß die ersten und die zweiten optischen Fibermittel von einer Ummantelung (13) umgeben sind; die zweiten optischen Fibermittel (37, 39) eine Mehrzahl Fiber zum Übertrgen des Bezugslichtstrahles an die und von den Reflektormittel(n) (45) umfassen; die Reflektormittel von den Wandlermitteln (19) an der entfernten Stelle getrennt sind und die entfernten Enden der Mehrzahl Fiber der zweiten optischen Fibermittel (37, 39) umfassen; die lichtempfindlichen Mittel ein erstes lichtempfindliches Element (25) zum Erzeugen des Meßsignales und ein zweites lichtempfindliches Element (41) zum Erzeugen desjenigen Signales umfassen, das die Intensität des Bezugslichtstrahles, welcher mittels der zweiten optischen Fibermittel (37, 39) zurückkommt, anzeigt; und die Korrekturmittel (37, 39, 41, 45 bis 47, 49) kontinuierlich die Intensität des Bezugslichtstrahles und des Meßlichtstrahles einstellen, wobei die Intensität des Bezugslichtstrahles entsprechend Signal von dem zweiten lichtempfindlichen Element (41) derart eingestellt wird, daß der Bezugslichtstrahl, welcher mittels der zweiten optischen Fibermittel zurückkommt, eine im wesentlichen konstante Intensität hat.

2. Optische Wandlervorrichtung nach Anspruch 1, wobei die vorbestimmte zu messende Variable der Druck eines Fluids ist; die Wandlermittel ein lichtreflektierendes Diaphragma (21) umfassen, das entsprechend dem Druck des Fluides bewegbar ist; und die ersten optischen Fibermittel eine Emitterfiber (15) zum Richten des Lichtstrahles auf das lichtreflektierende Diaphragma der Wandlermittel und eine Rückfiber (17) zum Empfangen des von dem Diaphragma reflektierten Lichtes beinhalten, wobei das Verhältnis des Lichtstrahles, der von dem Diaphragma reflektiert und der von der Rückfiber empfangen wird, sich entsprechend der Bewegung des Diaphragmas ändert, wodurch das Meßsignal den Druck des Fluides anzeigt.

3. Optische Wandlervorrichtung nach Anspruch 1, wobei die ersten optischen Fibermittel eine Emitterfiber (15) zum Übertragen des Lichtstrahles an die entfernte Stelle und eine Rückfiber (17) zum Übertragen des modulierten Lichtstrahles von der entfernten Stelle an das erste lichtempfindliche Element umfassen; und ein begrenztes Biegen der ersten optischen Fibermittel den Transmissionsfaktor der Emitterfiber und der Rückfiber verändert.

4. Optische Wandlervorrichtung nach Anspruch 3, wobei die zweiten optischen Fibermittel eine Emitterfiber (37) zum Übertragen des Bezugslichtstrahles an die Reflektiermittel und eine Rückfiber (39) zum Rückübertragen des Lichtes von den Reflektiermitteln umfassen, wobei die Reflektiermittel (45) einen vorbestimmten Anteil des von der Emitterfiber abgegebenen Lichtes auf die Rückfiber richten.

5. Optische Wandlervorrichtung nach Anspruch 3 oder 4, die umfaßt: feste Abschlußmittel (75) und Anbringungsmittel (77, 78) zum Anbringen der entfernten Enden der Emitterfiber, der Rückfiber und der Ummantelung an den festen Abschlußmitteln; und einen Draht (85), der innerhalb der Ummantelung angeordnet und mittels der Anbringungsmittel an den festen Abschlußmitteln angebracht ist, wobei der Draht die auf die Emitterfiber, die Rückfiber und die Ummantelung wirkende Zugspannung reduziert.

6. Optische Wandlervorrichtung nach Anspruch 5, wobei die festen Abschlußmittel eine Hülse (75) umfassen; und die Anbringungsmittel eine Epoxyd-Werkstoff (77) innerhalb der Hülse beinhalten.

7. Optische Wandlervorrichtung nach Anspruch 6, wobei die vorbestimmte zu messende Variable der Druck eines Fluides ist; die Wandlermittel (19) ein lichtreflektierendes Diaphragma (21) umfassen mit einer ersten Oberfläche, welche eine Kammer (95) begrenzt, die zur Atmosphäre hin offen ist, und einer zweiten, entgegengesetzten Oberfläche, die mit dem Fluid kommuniziert; und der Epoxyd-Werkstoff (77) einen Port (97) zum Lüften der Kammer (95) umfaßt, die von der ersten Oberfläche des lichtreflektierenden Diaphragma begrenzt ist.

8. Optische Wandlervorrichtung nach Anspruch 6 oder 7, wobei die Hülse (75) jenseits der entfernten Enden der Emitterfiber (37) und der Rückfiber (39) der zweiten optischen Fibermittel angeordnet ist; und die Reflektiermittel der zweiten optischen Fibermittel transparente Epoxyd-Mittel (45) umfassen, welche die entfernten Enden der Emitterfiber (37) und der Rückfiber (39) der zweiten optischen Fibermittel miteinander verbinden.

9. Optische Wandlervorrichtung nach einem der Ansprüche 6 bis 8, wobei der Draht (85) innerhalb der Ummantelung (13) mittels des Epoxyd-Werkstoffes (77) fest an der Innenfläche der Hülse (75) gesichert ist, wobei der Draht kürzer sowohl als die Ummantelung als auch als die ersten optischen Fibermittel (15, 17) ist, um jeder auf die Vorrichtung wirkenden Zugspannung zu widerstehen.

10. Optische Wandlervorrichtung nach einem der vorangehenden Ansprüche, wobei die vorbestimmte zu messende Variable der Druck eines Fluides ist; und die Wandlermittel einen Balg (1) umfassen, der ein lichtreflektierendes Diaphragma (21) aufweist, das mit Abstand den entfernten Enden der Emitterfiber (15) und der Rückfiber (17) der ersten optischen Fibermittel gegenüberliegt, wobei de Balg bezüglich der Emitter- und der Rückfiber entsprechend dem Druck des Fluides beweglich ist.

**Revendications**

1. Transducteur optique comprenant: une source de lumière (23); des premiers moyens à fibres optiques (15, 17) couplés à la source de lumière pour transmettre un faisceau lumineux de messure à destination de et en provenance d'une emplacement éloigné, ces premiers moyens à fibres optiques comprenant plusieurs fibres et ayant une transmittance lumineuse variable; un moyen de transduction (19) situé sur l'emplacement éloigné pour moduler l'intensité du faisceau lumineux des premiers moyens à fibres optiques en fonction de l'amplitude d'une variable prédéterminée à mesurer; des photocapteurs (25, 41) destinés à produire un signal de mesure indicatif de l'intensité du faisceau lumineux transmis à partir de l'emplacement éloignée par les premiers moyens à fibres optiques; un modulateur (51) destiné à mettre sous tension par intermittence la source de lumière à une vitesse déterminée par un oscillateur (63) pour produire un signal de mesure intermittent de façon correspondante; et des moyens de correction (37, 39, 41, 45 à 47, 49) synchronisés avec le modulateur (51) et reliés à la source de lumière (23) pour ajuster l'intensité du faisceau lumineux de mesure afin de corriger le signal de mesure pour tenir compte des variations de la transmittance des premiers moyens à fibres optiques (15, 17), les moyens de correction comprenant des seconds moyens à fibres optiques (37, 39) qui servent à transmettre un faisceau lumineux de référence au moyen réfléchissant (45) à l'emplacement éloigné, les moyens photocapteurs (25, 41) fournissant un signal indicatif de l'intensité du faisceau lumineux de référence transmis à partir de l'emplacement éloigné; caractérisé en ce que: les premiers et seconds moyens à fibres optiques sont entourés d'une gaine (13); les seconds moyens a fibres optiques (37, 39) comprennent plusieurs fibres destinées à transmettre le faisceau lumineux de référence à destination de et en provenance du moyen réfléchissant (45); le moyen réfléchissant est séparé du moyen de transduction (19) à l'emplacement éloignée et renforme les extrémité éloignées de la pluralité de fibres des seconds moyens à fibres optiques (37, 39);

le moyen photocapteur comprend une premier élément photocapteur (25) destiné à produire le signal de mesure et un second élément photocapteur (41) destiné à produire le signal indicatif de l'intensité du faisceau lumineux de référence renvoyé par les seconds moyens à fibres optiques (37, 39); et les moyens de correction (37, 39, 41, 45 à 47, 49) ajustent en continu l'intensité du faisceau lumineux de référence et du faisceau lumineux de mesure, l'intensité du faisceau lumineux de référence étant ajustée en fonction du signal provenant du second élément photocapteur (41) de sorte que le faisceau lumineux de référence renvoyé par les seconds moyens à fibres optiques présente une intensité sensiblement constante.

2. Transducteur optique selon la revendication 1, dans lequel la variable prédéterminée à mesurer est la pression d'une fluide; le moyen de transduction comprend une membrane réfléchissante à la lumière (21) qui est mobile en fonction de la pression du fluide; et les premiers moyens à fibres optiques comprennent une fibre émettrice (15) destinée à diriger le faisceau lumineux au niveau de la membrane réfléchissante à la lumière du moyen de transduction et une fibre de renvoi (17) destinée à recevoir la lumière réfléchie par la membrane, la proportion du faisceau lumineux qui est réfléchie par la membrane et reçue par la fibre de renvoi variant en fonction du mouvement de la membrane, de telle sorte que le signal de mesure est indicatif de la pression du fluide.

3. Transducteur optique selon la revendication 1, dans lequel les premiers moyens à fibres optiques comprennent une fibre émettrice (15) destinée à transmettre le faisceau lumineux à l'emplacement éloigné, et une fibre de retour (17) destinée à transmettre le faisceau lumineux modulé en provenance de l'emplacement éloigné à destination du premier élément photocapteur; et une flexion limitée des premiers moyens à fibres optiques fait varier la transmittance de la fibre d'émetteur et de la fibre de retour.

4. Transducteur optique selon la revendication 3, dans lequel les seconds moyens à fibres optiques comprennent une fibre émettrice (37) destinée à transmettre le faisceau lumineux de référence au moyen réfléchissant et une fibre de renvoi (39) destinée à renvoyer la lumière provenant du moyen réfléchissant, le moyen réfléchissant (45) dirigeant une proportion prédéterminée de la lumière fournie par la fibre émettrice à destination de la fibre de renvoi.

5. Transducteur optique selon la revendication 3 ou 4, comprenant des moyens de terminaison rigide (75) et des moyens de fixation (77, 78) destinés à fixer les extrémités éloignées de la fibre émettrice, de la fibre de renvoi et de la gaine sur le moyen de terminaison rigide; et une fil (85) située à l'intérieur de la gaine et assujetti par le moyen de fixation sur la terminaison rigide, le fil réduisant l'effort de traction appliqué à la fibre émettrice, à la fibre de renvoi et à la gaine.

6. Transducteur optique selon la revendication 5, dans lequel la terminaison rigide comprend un manchon (75); le moyen de fixation comprend une matière époxyde (77) située à l'intérieur du manchon.

7. Transducteur optique selon la revendication 6, dans lequel la variable prédéterminée à mesurer est la pression d'un fluide; le moyen de transduction (19) comprend une membrane réfléchissant la lumière (21) ayant une première surface limitrophe d'une chambre (95) donnant à l'atmosphère et une seconde surface en regard communiquant avec le fluide; et la matière époxyde (77) comprend un orifice (97) destiné à aérer la chambre (95) limitrophe de la première surface de la membrane réfléchissant la lumière.

8. Transducteur optique selon la revendication 6 ou 7, dans lequel le manchon (75) est situé au-delà des extrémités éloignées de la fibre émettrice (37) et de la fibre de renvoi (39) des seconds moyens à fibres optiques; et le moyen réfléchissant des seconds moyens à fibres optiques comprend un moyen époxyde translucide (45) assujettissant conjointement les extrémités éloignées de la fibre d'émetteur (37) et de la fibre de renvoi (39) des seconds moyens à fibres optiques.

9. Transducteur optique selon l'une quelconque des revendications 6 à 8, dans lequel le fil (85) situé à l'intérieur de la gaine (13) est fixé de façon rigide sur la surface intérieure du manchon (75) par la matière époxyde (77), le fil étant de longueur plus courte que la gaine et les premiers moyens à fibres optiques (15, 17) pris ensemble pour résister à l'effort de traction appliqué à l'appareil.

10. Transducteur optique selon l'une quelconque des revendications précédentes, dans lequel la variable prédéterminée à mesurer est la pression d'un fluide; et le moyen de transduction comprend un soufflet (19) ayant une membrane réfléchissant la lumière (21) limitrophe en relation espacée des extrémités éloignées de la fibre émetrice (15) et de la fibre de renvoi (17) des premiers moyens à fibres optiques, le soufflet étant mobile par rapport aux fibres émettrices et de renvoi en fonction de la pression du fluide.

Fig.1

Fig.5

Fig. 2

Fig. 3

Fig. 4